# EUROPEAN PATENT APPLICATION

(11) **EP 1 867 730 A1**
(43) Date of publication of application: **19.12.2007**
(21) Application number: 06730548.2
(22) Date of filing: 23.03.2006
(51) Int. Cl.: C12P 41/00, C07D 233/76, C07D 233/78, C12N 15/09, C07B 61/00

(54) **PROCESS FOR PRODUCTION OF OPTICALLY ACTIVE HYDROXYMETHYL -SUBSTITUTED PHENYLALANINE**

(30) Priority: 23.03.2005 JP 2005084850
(71) Applicant: Ajinomoto Co., Inc., Tokyo 104-8315 (JP)
(72) Inventor: NOZAKI, Hiroyuki, -ku, Kawasaki-shi, Kanagawa, 2108681 (JP); HASHIMOTO, Atsuko, -ku, Kawasaki-shi, Kanagawa, 2108681 (JP); NAKAZAWA, Masakazu, -ku, Kawasaki-shi, Kanagawa, 2108681 (JP)
(74) Representative: Nicholls, Kathryn Margaret
(86) International application number: PCT/JP2006/306600
(87) International publication number: WO 2006/101266

(57) **Abstract**

The present invention provides a production method of optically active hydroxymethyl-substituted phenylalanine, which includes reducing cyano-substituted benzylidene hydantoin (1) to give aminomethyl-substituted benzyl hydantoin (2) or a salt thereof, converting an amino group of the aminomethyl-substituted benzyl hydantoin (2) or a salt thereof to a hydroxyl group to give hydroxymethyl-substituted benzyl hydantoin (3), treating the hydroxymethyl-substituted benzyl hydantoin (3) with an enzyme to give D-hydroxymethyl-substituted phenylalanine (4a) or a salt thereof, or L-hydroxymethyl-substituted phenylalanine (4b) or a salt thereof.

According to the present invention, a production method capable of conveniently producing optically active hydroxymethyl-substituted phenylalanine on an industrial scale can be provided.

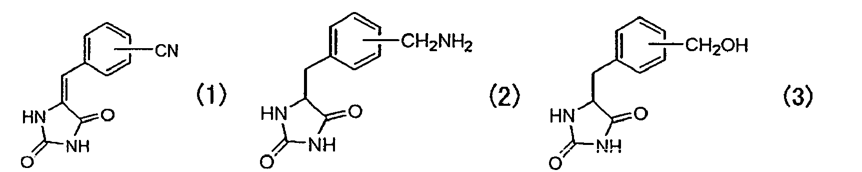

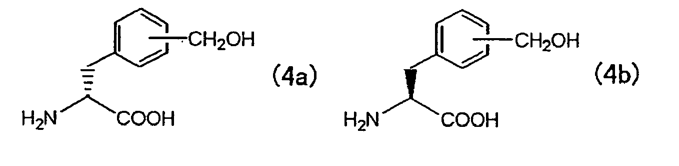

## Description

### Technical Field

The present invention relates to a production method of optically active hydroxymethyl-substituted phenylalanine.

### Background Art

Optically active hydroxymethyl-substituted phenylalanine derivative is a compound useful as a pharmaceutical agent or a starting material thereof. For example, an L form of 4-hydroxymethyl phenylalanine is used as an antihypertensive drug (WO92/14706), and a D form thereof is used as an intermediate for bradykinin B1 receptor antagonist and the like (JP-A-2004-525936).

As a conventional production method of 4-hydroxymethyl phenylalanine, for example, a method comprising, using ethyl 4-bromomethylbenzoate as a starting material, reduction, acetylation, condensation with diethyl acetamidomalonate, hydrolysis and decarboxylation, as shown in the following reaction scheme, to give N-acetyl-4-hydroxymethyl phenylalanine, which is followed by hydrolysis of N-acetyl-4-hydroxymethyl phenylalanine with acylase to give L-(4-hydroxymethyl)phenylalanine is known (Biochimica et Biophysica Acta, (1967), 148(2), 414-422). However, the starting material is expensive and difficult to obtain in large amounts, and 4-hydroxymethylbenzyl bromide of the intermediate is unstable, which renders a large-scale production difficult. In addition, hydrolysis with acylase by-produces unnecessary acetylated form (D form), making the yield only 50% at maximum.

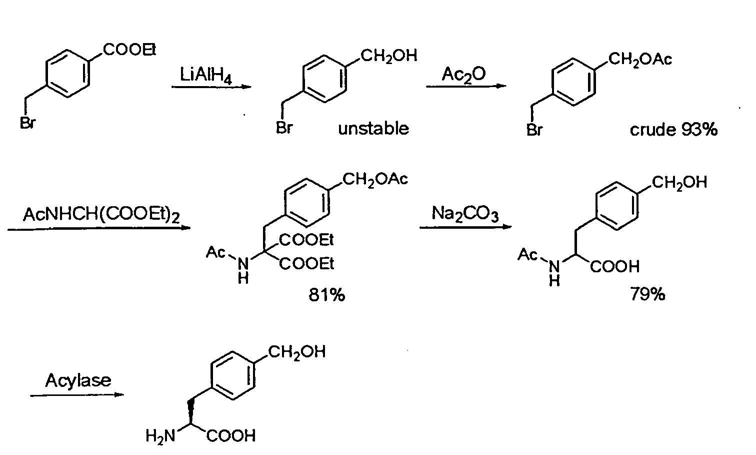

Moreover, according to the production method wherein 4-hydroxymethyl phenylalanine is obtained by alkylation of diphenylmethyleneglycine ester using an optically active phase-transfer catalyst, the ratio of an L form and a D form (L/D) is 4/1, showing poor stereoselectivity, which necessitates further purification (Int. J. Peptide Protein Res., (1994), 44, 457-465). Furthermore, since a production method wherein the 4-position of optically active 4-iodophenylalanine derived from optically active phenylalanine or the 4-position of optically active tyrosine trifluorate derivative derived from optically active tyrosine is converted to aldehyde, and then reduced to give optically active 4-hydroxymethyl phenylalanine uses expensive trioctylsilane and 1,3-bis(diphenylphosphino)propane, it is unsuitable for large-scale synthesis (Synthetic Communications, (1998), 28(2), 4279-4285).

As discussed above, conventional production methods of optically active hydroxymethyl-substituted phenylalanine are associated with problems in that expensive reagent is indispensable, the obtained intermediate is unstable, the yield is low, purification is needed since the purity is low and the like. Thus, they are practically unsuitable for industrial scale production. Therefore, development of a production method capable of conveniently obtaining optically active hydroxymethyl-substituted phenylalanine on an industrial scale is desired.

The present invention has been made in consideration of such actual situation wherein the problem to be solved is provision of a production method capable of conveniently obtaining optically active hydroxymethyl-substituted phenylalanine on an industrial scale; a compound useful for the production method and a method of producing the compound.

### Disclosure of the Invention

The present inventors have conducted intensive studies in an attempt to solve the above-mentioned problems and found that optically active hydroxymethyl-substituted phenylalanine can be conveniently obtained in a high yield by reduction and hydroxylation of cyano-substituted benzylidene hydantoin as a starting material and treatment of the resulting hydroxymethyl-substituted benzylhydantoin with an enzyme, which resulted in the completion of the present invention.

Accordingly, the present invention is characterized by the following.
[1] A method of producing optically active hydroxymethyl-substituted phenylalanine comprising reducing cyano-substituted benzylidene hydantoin represented by the following formula (1) 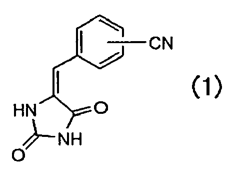 to give aminomethyl-substituted benzyl hydantoin represented by the following formula (2) 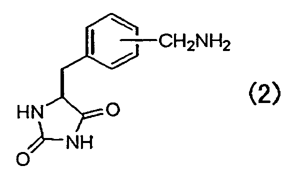 or a salt thereof, converting an amino group of the aminomethyl-substituted benzyl hydantoin or a salt thereof to a hydroxyl group to give hydroxymethyl-substituted benzyl hydantoin represented by the following formula (3) 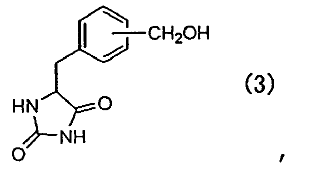 and treating the hydroxymethyl-substituted benzylhydantoin with an enzyme to give D-hydroxymethyl-substituted phenylalanine represented by the following formula (4a) 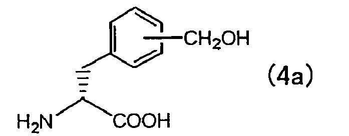 or a salt thereof, or L-hydroxymethyl-substituted phenylalanine represented by the following formula (4b) 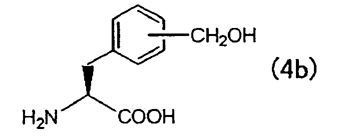 or a salt thereof.
[2] The method of [1], wherein the reduction is performed by hydrogenation in the presence of a reduction catalyst.
[3] The method of [2], wherein the reduction catalyst is a transition metal catalyst.
[4] The method of [2], wherein the reduction catalyst is a palladium catalyst.
[5] The method of any one of [1]-[4], wherein the amino group is converted to a hydroxyl group by reaction with a nitrite salt.
[6] The method of any one of [1]-[5], wherein the enzyme is at least one kind selected from the group consisting of hydantoin racemase, hydantoinase and carbamoylase.
[7] The method of any one of [1]-[5], wherein the enzyme is at least one kind selected from the group consisting of hydantoin racemase derived from Microbacterium liquefaciens AJ3912 strain, hydantoinase derived from Flavobacterium sp. AJ11199 strain and carbamoylase derived from Flavobacterium sp. AJ11199 strain.
[8] The method of any one of [1]-[5], wherein the enzyme is obtained from a culture of a microorganism transformed with a recombinant DNA.
[9] A method of producing aminomethyl-substituted benzyl hydantoin represented by the following formula (2) 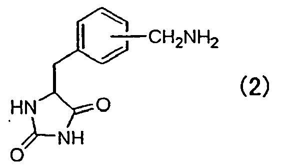 or a salt thereof, which comprises reducing cyano-substituted benzylidene hydantoin represented by the following formula (1) 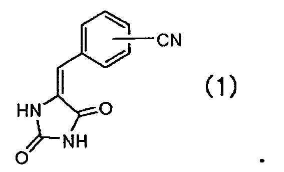
[10] The method of [9], wherein the reduction is performed by hydrogenation in the presence of a reduction catalyst.
[11] The method of [10], wherein the reduction catalyst is a transition metal catalyst.
[12] The method of [10], wherein the reduction catalyst is a palladium catalyst.
[13] A method of producing hydroxymethyl-substituted benzyl hydantoin represented by the following formula (3) 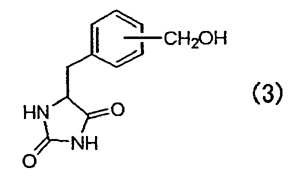 which comprises converting an amino group of aminomethyl-substituted benzyl hydantoin represented by the following formula (2) 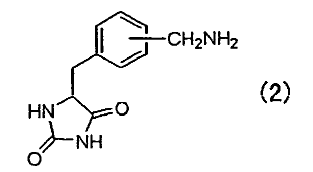 or a salt thereof to a hydroxyl group.
[14] The method of [13], wherein the amino group is converted to a hydroxyl group by reaction with a nitrite salt.
[15] A method of producing optically active hydroxymethyl-substituted phenylalanine, which comprises treating hydroxymethyl-substituted benzyl hydantoin represented by the following formula (3) 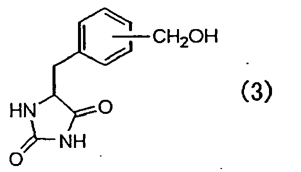 with an enzyme to give D-hydroxymethyl-substituted phenylalanine represented by the following formula (4a) 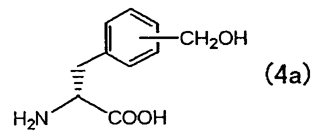 or a salt thereof, or L-hydroxymethyl-substituted phenylalanine represented by the following formula-(4b) 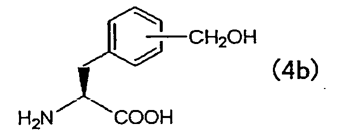 or a salt thereof.
[16] The method of [15], wherein the enzyme is at least one kind selected from the group consisting of hydantoin racemase, hydantoinase and carbamoylase.
[17] The method of [15], wherein the enzyme is at least one kind selected from the group consisting of hydantoin racemase derived from Microbacterium liquefaciens AJ3912 strain, hydantoinase derived from Flavobacterium sp. AJ11199 strain and carbamoylase derived from Flavobacterium sp. AJ11199 strain.
[18] The method of [15], wherein the enzyme is obtained from a culture of a microorganism transformed with a recombinant DNA.
[19] Aminomethyl-substituted benzyl hydantoin represented by the following formula (2) 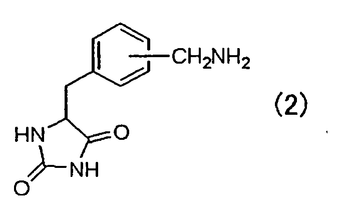 or a salt thereof.
[20] Hydroxymethyl-substituted benzyl hydantoin represented by the following formula (3)

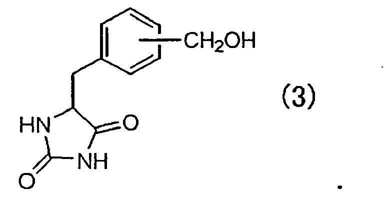

### Best Mode for Embodying the Invention

The present invention is explained in detail in the following by referring to a preferable embodiment thereof.

The production method of the present invention is shown in the following reaction scheme.

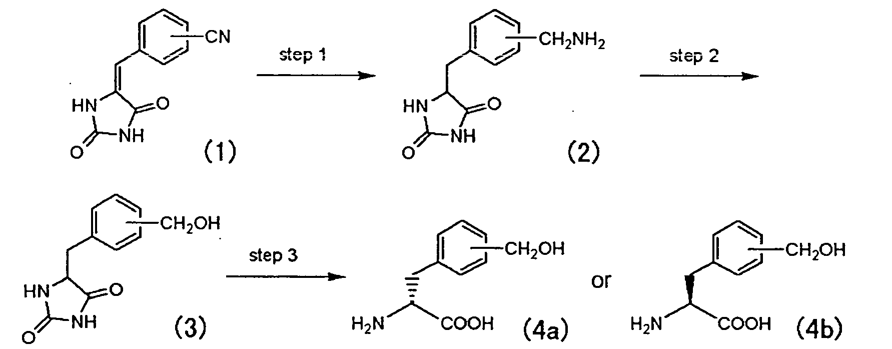

### (Step 1)

In Step 1, cyano-substituted benzylidene hydantoin represented by the above-mentioned formula (1) (hereinafter to be referred to as "benzylidene hydantoin (1)") is reduced to give aminomethyl-substituted benzyl hydantoin represented by the above-mentioned formula (2) or a salt thereof (hereinafter to be referred to as "benzyl hydantoin (2)"). For the reduction reaction here, it is preferable to add a reduction catalyst and acid to benzylidene hydantoin (1) and allow the mixture to react with hydrogen gas in a solvent. As the salt of benzyl hydantoin (2), an acid addition salt such as hydrochloride, hydrosulfate, trifluoroacetate and the like can be mentioned. The substitution position of the cyano group of benzylidene hydantoin (1) may be either of the 2-position, the 3-position and the 4-position, with preference given to the 4-position.

The reduction catalyst is not particularly limited as long as it can be used for hydrogenation, and a transition metal catalyst is preferably used. As the transition metal catalyst, palladium catalysts such as palladium, palladium carbon, palladium black, palladium chloride and the like, as well as platinum oxide, platinum black, Raney-nickel and Raney-cobalt can be recited as examples, of which palladium catalysts such as palladium, palladium carbon, palladium black, palladium chloride and the like are particularly preferable. The amount of the transition metal catalyst to be used is generally 0.1-5 mol%, preferably 0.5-2 mol%, relative to benzylidene hydantoin (1). As the acid, inorganic acids such as hydrochloric acid, sulfuric acid and the like are preferably used. The amount of the acid to be used is generally 1.0-1.5 equivalents, preferably 1.0-1.2 equivalents, relative to benzylidene hydantoin (1). As the solvent, aqueous solvent such as water, or a mixed solvent of methanol, ethanol and the like with water can be used. The reaction time is generally 1-12 hr, preferably 2-9 hr. After completion of the reaction, the transition metal catalyst is filtered off, and the reaction solvent is evaporated to give benzyl hydantoin (2). The obtained benzyl hydantoin (2) can be isolated and purified by a conventional method such as chromatography and the like. However, it can be used for the production of hydroxymethyl-substituted benzyl hydantoin represented by the above-mentioned formula (3) (hereinafter to be referred to as "benzyl hydantoin (3)") without isolation and purification.

### (Step 2)

In Step 2, the amino group of benzyl hydantoin (2) is converted to a hydroxyl group to give benzyl hydantoin (3). The aminomethyl group of benzyl hydantoin (2) may be substituted at either of the 2-position, the 3-position and the 4-position, with preference given to the 4-position.

The method of the above-mentioned conversion is not particularly limited as long as it can convert the amino group to a hydroxyl group. It is preferably performed by reaction with a nitrite salt. As the nitrite salt, alkali metal nitrite such as sodium nitrite, potassium nitrite and the like can be used, with preference given to sodium nitrite. The amount of the nitrite salt to be used is generally 1.0-1.5 equivalents, preferably 1.2-1.3 equivalents, relative to benzyl hydantoin (2). The reaction temperature is generally 95-100°C, preferably 98-100°C. The reaction time is generally 15-24 hr, preferably 18-22 hr. The above-mentioned reaction may be carried out in a solvent. As the solvent, water, ethanol/dimethylformamide and water/dimethylformamide can be mentioned, with preference given to water. After completion of the reaction, the reaction mixture is concentrated to dryness to give benzyl hydantoin (3).

### (Step 3)

In Step 3, benzyl hydantoin (3) is treated with an enzyme to give optically active hydroxymethyl-substituted phenylalanine or a salt thereof (hereinafter to be referred to as "phenylalanine (4)"). As a result, D-hydroxymethyl-substituted phenylalanine represented by the following formula (4a) or a salt thereof (hereinafter to be referred to as "D-phenylalanine (4a)") or L-hydroxymethyl-substituted phenylalanine represented by the following formula (4b) or a salt thereof (hereinafter to be referred to as "L-phenylalanine (4b)") can be conveniently obtained from benzyl hydantoin (3) optically selectively in a high yield. As the salt of phenylalanine (4), D-phenylalanine (4a) and L-phenylalanine (4b), an acid addition salt such as hydrochloride, hydrogen bromide, hydrosulfate, phosphate and the like, or an alkali metal salt such as sodium salt, potassium salt and the like, an alkaline earth metal salt such as calcium salt and the like, an ammonium salt and the like can be recited as examples, and hydrochloride and sodium salt can be particularly mentioned.

The enzyme is not particularly limited as long as it can optically selectively hydrolyze benzyl hydantoin (3) to give optically active phenylalanine (4), and conventionally known enzymes derived from microorganisms such as bacteria, actinomycetes, fungi and the like or plant and animal can be used. As such enzyme, for example, hydantoin racemase, hydantoinase and carbamoylase can be mentioned, which may be used alone or in combination of two or more kinds thereof. When two or more kinds thereof are used in combination, preferable combinations of enzymes are as follows.
(i) hydantoinase and carbamoylase
(ii) hydantoinase, carbamoylase and hydantoin racemase

When two or more kinds of enzymes are used in combination, for example, benzyl hydantoin (3) is reacted with hydantoinase having optical selectivity to give either of L-(N-carbamoyl)amino acid and D-(N-carbamoyl)amino acid, and carbamoylamino acid is further reacted with carbamoylase to give optically active phenylalanine.

Even when hydantoinase to be used does not have optical selectivity, optically active amino acid can be obtained using carbamoylase having optical selectivity. That is, since hydantoinase catalyzes not only hydrolyzing reaction but also dehydrating condensation reaction, an amino acid having desired steric configuration can be obtained by using hydantoin racemase and carbamoylase having optical selectivity in combination.

When D-phenylalanine is to be produced, at least one kind selected from the group consisting of hydantoin racemase derived from Microbacterium liquefaciens AJ3912 strain, D-hydantoinase derived from Flavobacterium sp AJ11199 strain and D-carbamoylase derived from Flavobacterium sp AJ11199 strain is desirably used.

When L-phenylalanine is to be produced, at least one kind selected from the group consisting of hydantoin racemase derived from Microbacterium liquefaciens AJ3912 strain, L-hydantoinase derived from Flavobacterium sp AJ11199 strain and L-carbamoylase derived from Flavobacterium sp AJ11199 strain is desirably used.

Microbacterium liquefaciens AJ3912 strain was first deposited as Flavobacterium sp. AJ3912 (FERM-P3133) strain on June 27, 1975 at the National Institute of Bioscience and Human-Technology, Agency of Industrial Science and Technology, Ministry of International Trade and Industry but, as a result of re-identification, clarified to belong to Aureobacterium liquefaciens. At present, due to change of species name, Aureobacterium liquefaciens is classified in Microbacterium liquefaciens and deposited as Microbacterium liquefaciens AJ3912 (domestic depositary No. FERM-P3133, international depositary No. FERM-BP7643) at the International Patent Organism Depositary, National Institute of Advanced Industrial Science and Technology.

In addition, Flavobacterium sp. AJ11199 (FERM-P4229) strain is a microorganism first deposited as Alcaligenes aquamarinus at the National Institute of Bioscience and Human-Technology, Agency of Industrial Science and Technology, Ministry of International Trade and Industry but, as a result of re-identification, clarified to belong to Flavobacterium sp. At present, it is deposited as Flavobacterium sp. AJ11199 (FERM-P4229) strain at the International Patent Organism Depositary, National Institute of Advanced Industrial Science and Technology.

For the enzyme reaction, an enantiomer mixture (e.g., racemate) of benzyl hydantoin (3) to be a substrate is desirably treated under the following conditions in consideration of the enzyme activity. As the reaction solvent, an aqueous medium such as water (e.g., ion exchange water), a buffer and the like can be generally used. As long as the enzyme reaction is not inhibited, an organic solvent such as alcohol and the like may be contained to the reaction solvent. The reaction temperature is generally 20°C to 50°C, preferably 30°C to 40°C. While the reaction pH depends on the optimal pH of the enzyme, neutral condition is desirable, preferably pH 5.0-9.0, more preferably 6.0-8.0. The reaction time is generally 15-48 hr, preferably 20-30 hr. The amount of the enzyme to be used is not particularly limited, but those of ordinary skill in the art can appropriately determine the amount with ease by performing a preliminary experiment under respective reaction conditions. After the enzyme reaction, the reaction mixture is acidified (e.g., pH 2) to deactivate the enzyme, treated with activated carbon etc. and filtered to remove the enzyme.

The aforementioned enzyme may be obtained from a culture of a microorganism transformed with a recombinant DNA inserted an enzyme gene such as hydantoinase and the like (hereinafter to be referred to as "transformant").

The recombinant DNA can be obtained, for example, by ligating a DNA fragment wherein a promoter, a DNA encoding enzyme such as hydantoinase and the like and a terminator are ligated in this order, and a vector DNA.

The promoter is not particularly limited as long as it can promote transcription of object enzyme gene, and a promoter generally used for production of heterologous protein in Escherichia coli can be used. For example, T7 promoter, trp promoter, lac promoter, tac promoter, PL promoter and the like can be mentioned.

As the DNA encoding the enzyme, for example, one isolated from chromosomal DNA of Flavobacterium sp. AJ11199 strain and one isolated from chromosomal DNA of Microbacterium liquefaciens AJ3912 strain can be used. For isolation of DNA from microorganism, for example, a conventional method such as disruption by ultrasonication, ethanol precipitation, column chromatography and the like can be used. As such DNA, two or more kinds of DNAs can be used. For example, a recombinant DNA having 3 genes of hydantoinase, carbamoylase and hydantoin racemase is used. Thus, by simultaneous expression of 3 enzymes using one transformant, phenylalanine (4) can be produced. Therefore, it is efficient as compared to independent use of plural transformants.

To increase the production amount, a terminator, which is a transcription terminator sequence, is preferably linked at the downstream of an enzyme gene. As such terminator, rrnB terminator, T7 terminator, fd terminator, T4 terminator, terminator of tetracycline resistant gene, terminator of Escherichia coli trpA gene and the like can be mentioned. Of these, rrnB terminator and the like are preferable because they improve stability of plasmid.

A vector DNA to be ligated to a DNA fragment, wherein a DNA encoding enzyme such as hydantoinase and the like is ligated, for preparation of a recombinant DNA to be introduced into a host cell, is preferably of a multi copy type. Examples thereof include one a plasmid having a replication origin derived from Col E1, such as pUC series plasmids, pBR322 series plasmids, or derivatives thereof. Here, the derivative means a plasmid obtained by alteration such as substitution, deletion, insertion, addition or inversion of base and the like. The alteration includes, besides the aforementioned substitution of base and the like, alteration by a mutation treatment with a mutating agent, UV irradiation and the like or an inartificial treatment and the like. For selection of a transformant, it is preferable that the vector have a marker such as ampicillin resistance gene, kanamycin resistance gene and the like. For ligation of genes, for example, the object gene amplified by PCR and the like and the multicloning site of vector DNA are cleaved with the same restriction enzyme, and the genes are ligated using, for example, a ligation kit and the like.

Then, the obtained recombinant DNA is introduced into a host cell to give a transformant. As the host cell, bacterial cell, actinomycetal cell, yeast cell, mycotic cell, plant cell, animal cell and the like can be used. Since there are a number of findings relating to the mass production of protein, Escherichia coli (enteric bacterium) is preferable, and Escherichia coli is more preferable. Particularly, Escherichia coli JM109 strain (more preferably, DE3) is preferable.

Then, the obtained transformant is cultured in a medium. When a host cell is Escherichia coli, the medium is, for example, a medium generally used for cultivating Escherichia coli, such as M9-casamino acid medium, LB medium and the like. The culture conditions and production-inducing conditions can be appropriately determined according to the kind of the marker of a vector, host fungus and the like.

After recovery of microorganism by centrifugation, the microorganism is disrupted or lysed, and the produced enzyme is recovered. The recovered enzyme can be used as a crude enzyme solution. As a method for disruption, for example, disruption by ultrasonication, disruption by French Press, disruption by glass beads and the like can be utilized. As a method for lysis, for example, egg-white lysozyme, peptidase treatment or a combination of these methods can be used. Where necessary, these enzymes can be purified by a general method of precipitation, filtration, column chromatography and the like before use. In this case, a purification method using an antibody of such enzyme can also be utilized.

For various operations dealing with plasmid, DNA fragment, various enzymes, preparation of transformant, selection of transformant and the like, the methods described in MOLECULAR CLONING, 2nd Edition, J. Sambrook et al, 1989, COLD SPRING HARB OR LABORATORY PRESS and the like can be applied.

Accordingly, for example, when D-phenylalanine (4a) is to be produced, a DNA encoding hydantoin racemase is introduced into a host cell harboring a DNA encoding D-hydantoinase and a DNA encoding D-carbamoylase to give a transformant, and an enzyme obtained by cultivating the transformant can be used. When L-phenylalanine (4b) is to be produced, a DNA.encoding hydantoin racemase is introduced into a host cell introduced a DNA encoding L-hydantoinase and a DNA encoding L-carbamoylase to give a transformant, and an enzyme obtained by cultivating the transformant can be used. In addition, an enzyme gene of Arthrobacter·aurescens as described in J. Biotechnol. 86, 19-30, 2001 can be used. Furthermore, variant hydantoinase of Arthrobacter·species as described in Nature Biotechnol. 18, 317-320, 2000 may also be combined.

Then, optically active phenylalanine (4) can be produced by treating benzyl hydantoin (3) with an enzyme obtained from a culture. For enzyme treatment, benzyl hydantoin (3) may be directly added to a medium containing the aforementioned transformant to give optically active phenylalanine (4), or the above-mentioned transformant may be directly added to a reaction mixture containing benzyl hydantoin (3). The composition and the like of the reaction mixture are not particularly limited as long as the enzyme reaction of benzyl hydantoin (3) proceeds.

The transformant can be prepared, for example, by a method similar to the method described in Example 8 of WO03/085108. In this case, the transformant only needs to be stood at the aforementioned temperature and pH for 8 hr-6 days. Optically active phenylalanine (4) accumulated in a culture medium or a reaction mixture can be recovered from the culture medium or the reaction mixture by a conventional method. For example, an appropriate combination of operations such as filtration, centrifugation, vacuum concentration, ion exchange or adsorption chromatography, crystallization and the like can be used as necessary.

Optically active phenylalanine (4) in the culture medium or the reaction mixture can be quickly quantified by a well-known method. For example, thin layer chromatography and high performance liquid chromatography (HPLC) using optical resolution column can be used.

Benzylidene hydantoin (1) can be produced, for example, by a method similar to the method described in US-B-(US2861079). Specifically, as shown in the following reaction scheme, ethanolamine is added to cyano-substituted benzaldehyde and hydantoin in water and the mixture is heated. Then, precipitated crystals were separated to give benzylidene hydantoin (1).

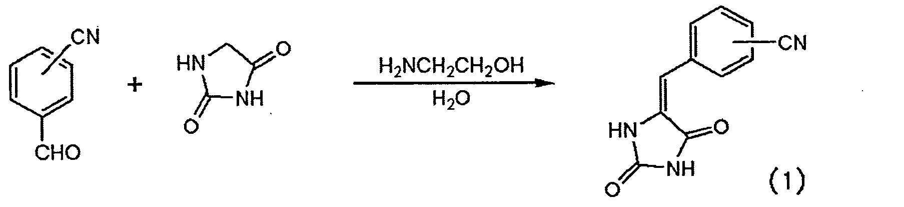

Optically active hydroxymethyl-substituted phenylalanine obtained by the aforementioned production method can be isolated and purified by a conventional method such as chromatography and the like, but can also be subjected to the next reaction without isolation and purification. For example, optically active N-t-butoxycarbonyl-4-hydroxymethyl phenylalanine methyl ester, which is a protected compound thereof, can be produced. Optically active N-t-butoxycarbonyl-4-hydroxymethyl phenylalanine methyl ester can be obtained by the method described in a literature (Bioorganic & Medicinal Chemistry (2000), 8(7), 1677-1696). Specifically, as shown in the following reaction scheme, methanol and thionyl chloride were mixed, optically active 4-hydroxymethyl phenylalanine was added to the mixture for esterification, and the mixture was reacted with sodium hydrogencarbonate and di-tert-butyldicarbonate to give optically active N-t-butoxycarbonyl-4-hydroxymethyl phenylalanine methyl ester. In the formula, the symbol * means an asymmetric carbon.

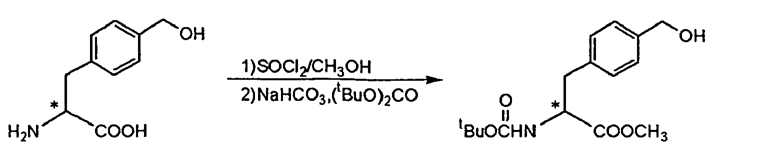

Furthermore, a hydroxyl group of the optically active N-t-butoxycarbonyl-4-hydroxymethyl phenylalanine methyl ester obtained by the above-mentioned production method is reacted with methanesulfonyl chloride based on the method described in the aforementioned JP-A-2004-525936 in a solvent such as dichloromethane, tetrahydrofuran and the like in the presence of a base such as triethylamine and the like, and then reacted with an amine compound (e.g., 2,6-dimethylpiperidine) to give an intermediate important as a bradykinin B1 receptor antagonist.

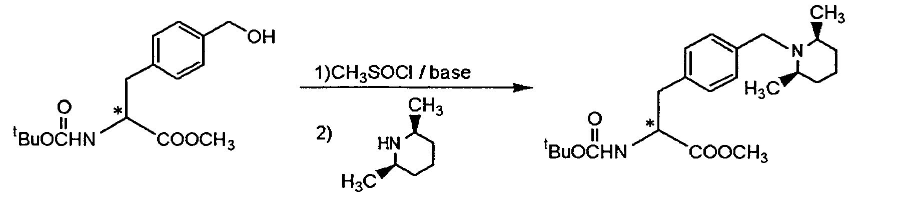

### Examples

The present invention is explained in detail in the following by referring to Examples, which are not to be construed as limitative.

### (Reference Example 1)

### Synthesis of 5-(4-cyanobenzylidene)hydantoin

Hydantoin (23.7 g, 0.23 mol) and 2-aminoethanol (5.4 ml, 0.09 mol) were added to water (250 ml), and the mixture was stirred with heating at 50°C. When the solution became transparent, 4-cyanobenzaldehyde (25.1 g, 0.18 mol) was added and the mixture was heated under reflux with stirring overnight. The reaction mixture was allowed to cool to room temperature and cooled with ice. The mixture was adjusted to pH 7 with hydrochloric acid and stirred for 1 hr. The slurry solution was filtered and dried under reduced pressure to give 5-(4-cyanobenzylidene)hydantoin (37.3 g). Yield 97.2%. ¹H-NMR(DMSO-d₆):δ6.45(s, 1H), 7.79(d, 2H), 7.85(d, 2H)
MS(ESI): 212.1[M-H]⁻

### (Example 1)

### Synthesis of 5-(4-aminomethylbenzyl)hydantoin hydrochloride

Under argon atmosphere, 5-(4-cyanobenzylidene)hydantoin (19.2 g, 0.09 mol) and hydrochloric acid (7.8 ml, 0.09 mol) were added to water (384 ml), and the mixture was stirred. 5% Palladium carbon (AD, 53.1% water) (16.4 g) was added to a slurry solution, the inside of the reaction container was substituted with hydrogen, and the mixture was vigorously stirred at room temperature for 9 hr. Palladium carbon was removed by filtration, and the mother liquor was concentrated under reduced pressure until precipitation of crystals. To remove remaining water, 2-butanol (50 ml) was added and the mixture was concentrated twice under reduced pressure. 2-Butanol (100 ml) was added to the residue, and the mixture was stirred for 2 hr. A slurry solution was filtered and dried under reduced pressure to give 5-(4-aminomethylbenzyl)hydantoin hydrochloride (16.1 g) as white crystals. Yield 69.8%.
¹H-NMR(DMSO-d₆):δ2.95(t, 2H), 3.97(s, 2H), 4.36(t, 1H), 7.21(d, 2H), 7.40(d, 2H)
¹³C-NMR(DMSO): 39.25, 39.46, 39.67, 39.88, 40.09, 40.30, 40.51, 58.58, 128.96, 130.35, 175.43
MS(ESI): 220.1[M+H]⁺, 218.3[M-H]⁻

### (Example 2)

### Synthesis of 5-(4-hydroxymethylbenzyl)hydantoin

5-(4-Aminomethylbenzyl)hydantoin hydrochloride (2.7 g, 10.6 mmol) and sodium nitrite (0.9 g, 12.7 mmol) were added to water (30 ml), and the mixture was heated under reflux with stirring overnight. The reaction mixture was allowed to cool to room temperature, adjusted to pH 2 with hydrochloric acid and the mixture was stirred for 1 hr. The reaction mixture was adjusted to pH 5 with 25% aqueous sodium hydroxide solution and the mixture was concentrated under reduced pressure. To remove remaining water, ethanol (30 ml) was added and the mixture was concentrated twice under reduced pressure. Ethanol (150 ml) was added to the residue, and the mixture was stirred. A slurry solution was filtered, and the mother liquor was concentrated under reduced pressure. 2-Propanol (15 ml) was added to the residue for slurry washing. The slurry solution was filtered and dried under reduced pressure to give 5-(4-hydroxymethylbenzyl)hydantoin (1.6 g). Yield 68.5%.
¹H-NMR(DMSO-d₆):δ2.91(d, 2H), 4.31(m, 1H), 4.45(d, 2H), 5.13(t, 1H), 7.12(d, 2H), 7.21(d, 2H), 7.91(s, 1H), 10.39(s, 1H)
¹³C-NMR(DMSO): 39.25, 39.46, 39.67, 39.88, 40.09, 40.30, 40.51, 58.77, 63.02, 126.54, 129.81
MS(ESI): 219.0 [M-H]-

### (Example 3)

### Synthesis of D-(4-hydroxymethyl)phenylalanine

5-(4-Hydroxymethylbenzyl)hydantoin (65.3 g, 0.30 mol) and manganese sulfate pentahydrate (0.4 g) were added to water (1770 ml), and the mixture was heated to 37°C. A microorganism suspension of E. coli D9 cell obtained by the method described in Example 8 of WO03/085108 was added to the aqueous solution to 4 g dried cell/L. The reaction mixture was adjusted to pH 7.2-7.5 with aqueous sodium hydroxide solution and acetic acid, and the mixture was stirred for 24 hr. The reaction mixture was adjusted to pH 2, 10% aqueous sanizol solution (9 ml) was added and the mixture was stirred at 55°C for 30 min. Activated carbon (Shirasagi Charcoal AW50: manufactured by Takeda Pharmaceutical Company Limited) (18 g) was added, and the mixture was further stirred for 1 hr. After celite filtration, the mother liquor was adjusted to pH 7 and concentrated under reduced pressure. To remove remaining water, 2-propanol (300 ml) was added and the mixture was further concentrated twice under reduced pressure. 2-Propanol (400 ml) was added to the residue, and the mixture was stirred, filtered and dried under reduced pressure to give D-(4-hydroxymethyl)phenylalanine (including sodium chloride) (106.9 g). By HPLC analysis, the content of D-(4-hydroxymethyl)phenylalanine was confirmed to be 47.0 wt% (yield 73.2%, optical purity not less than 99.0%ee). The optical purity was measured by HPLC under the following conditions.
HPLC measurement conditions for optical purity:
column: SUMICHIRAL OA-5000 (150 mm×4.6 mm)
eluent: 2 mM CuSO₄/IPA=95/5, UV: 254 nm, flow rate: 1.0 ml/min, temperature: room temperature
¹H-NMR(D₂O):δ3.21(m,2H),3.99(m,1H),4.64(s,2H),7.33(d,2H), 7.40(d, 2H) MS(ESI): 194.3 [M-H]⁻

### (Example 4)

### (1) Construction of hydantoinase, L-carbamoylase expression plasmid

Using genome DNA of Microbacterium liquefaciens AJ3912 strain as a template, primer HD_up (SEQ ID NO:1) and HD_down (SEQ ID NO:2), hydantoinase gene was amplified, treated with NdeI/BamHI, and ligate to pTrp4 treated with NdeI/BamHI in advance. E. coli JM109 was transformed with the ligation solution, and a strain having the object plasmid was selected from ampicillin resistant strains and the plasmid was named as pTrp3912H4.

Using pTrp3912H4 as a template, primer SDm2 (SEQ ID NO:3) and HD down (SEQ ID NO:2), hydantoinase gene containing SD sequence was amplified and ligate to pGEM-T easy (manufactured by Promega). E. coli JM109 was transformed with the ligation solution, and a strain having the object plasmid was selected from ampicillin resistant strains and the plasmid was named as pSD-H.

1.5kb of hydantoinase gene region (gene segment H) containing SD sequence which was obtained by treating pSD-H with EcoRI/BamHI, and 1.0 kb of gene segment (gene segment C) which was obtained by treating L-carbamoylase gene amplified using genome DNA of Microbacterium liquefaciens AJ3912 strain as a template, primer CH_up (SEQ ID NO:4) and CH_down (SEQ ID NO:5) with NdeI/EcoRI, were prepared. Gene segment C and gene segment A were ligated to pTrp8 treated with NdeI/BamHI, E. coli JM109 was transformed, and a transformant having the object plasmid was selected from chloramphenicol resistant strains and the plasmid was named as pTrp8C-SD-H.

Hydantoin racemase gene expression plasmid pTrp4R was introduced into E. coli JM109 having pTrp8C-SD-H to give E. coli L6 strain expressing genes encoding hydantoin racemase, hydantoinase and L-carbamoylase.

### (2) Preparation of enzyme microorganism

L6 strain was cultured on LB plate (10 g/L peptone, 5 g/L yeast extract, 10 g/L sodium chloride, 20 g/L agar) containing 50 mg/L ampicillin sodium and 25 mg/L chloramphenicol at 30°C for 20 hr. The cell was cultivated in LB liquid medium (10 g/L peptone, 5 g/L yeast extract, 10 g/L sodium chloride) containing 50 mg/L ampicillin sodium, 25 mg/L chloramphenicol and 20 mg/L cobalt sulfate pentahydrate with reciprocal shaking at 34°C for 16 hr. And then, the bacterial cells were collected by centrifugation (8,000 g, 10 min, 4°C).

### (3) Synthesis of L-(4-hydroxymethyl)phenylalanine

5-(4-Hydroxymethylbenzyl)hydantoin (0.1 g, 0.48 mmol) and cobalt chloride (1.3 mg) were added to 0.1M buffered aqueous potassium dihydrogen phosphate-dipotassium monohydrogen phosphate solution (pH 7.2, 3 ml), and the mixture was heated to 37°C. To this aqueous solution was added the E. coli L6 cell suspentionobtained in the aforementioned step (final concentration; 4 g dried cell/L), and the mixture was stirred for 6 days. The reaction mixture was adjusted to pH 2, 10% aqueous sanizol solution (0.02 ml) was added and the mixture was stirred at 55°C for 30 min. Activated carbon (Shirasagi Charcol AW50: manufactured by Takeda Pharmaceutical Company Limited, 40 mg) was added, and the mixture was further stirred for 1 hr. After celite filtration, the mother liquor was adjusted to pH 7 and concentrated under reduced pressure. To remove remaining water, 2-propanol (0.5 ml) was added and the mixture was further concentrated twice under reduced pressure. 2-Propanol (0.6 ml) was added to the residue, and the mixture was stirred, filtered and dried under reduced pressure to give 77 mg of L-(4-hydroxymethyl)phenylalanine (including sodium chloride). (yield 69.2%)

### (Example 5)

### Synthesis of D-(4-hydroxymethyl)phenylalanine methyl ester hydrochloride

D-(4-Hydroxymethyl)phenylalanine (4.3 g, 21.9 mmol) was added to methanol (43 ml), cooled to 5°C and stirred. Thionyl chloride (2.7 ml, 35.0 mmol) was slowly added dropwise, and the mixture was stirred at room temperature overnight. The reaction mixture was concentrated under reduced pressure, methanol (15 ml) was added again and the mixture was concentrated under reduced pressure. Ethyl acetate (20 ml) was added to the residue for slurry washing. The solution was filtered and dried under reduced pressure to give D-(4-hydroxymethyl)phenylalanine methyl ester hydrochloride (4.3 g). Yield 79.5%.
¹H-NMR(DMSO-d₆):δ3.12(m, 2H), 3.68(s, 3H), 4.25(t, 1H), 4.49(s, 2H), 7.19(d, 2H), 7.28(d, 2H)
MS(ESI): 210.3 [M+H]⁺

### (Example 6)

### D-(N-t-butoxycarbonyl)-(4-hydroxymethyl)phenylalanine methyl ester

D-(4-Hydroxymethyl)phenylalanine methyl ester hydrochloride (10.2 g, 41.1 mmol) was added to methanol (60 ml) and water (30 ml), and the mixture was stirred and adjusted to pH 8 with 25% aqueous sodium hydroxide solution. Di-t-butyl dicarbonate (13.6 g) was dissolved in methanol (10 ml) and added thereto. The mixture was maintained at pH 8 or above and stirred at room temperature overnight. The reaction solution was concentrated under reduced pressure. Water (50 ml) and ethyl acetate (100 ml) were added to the residue, and the mixture was stirred, and the insoluble material was filtered off. The mother liquor was separated and the ethyl acetate layer was washed with 0.5N hydrochloric acid, saturated aqueous sodium hydrogencarbonate solution, water and saturated brine (each 100 ml). The ethyl acetate solution was concentrated under reduced pressure, heptane (50 ml) was added and concentrated again under reduced pressure. Heptane (100 ml) was added to the residue and the mixture was stirred overnight. The slurry solution was filtered and dried under reduced pressure to give D-(N-t-butoxycarbonyl)-(4-hydroxymethyl)phenylalanine methyl ester (10.2 g). Yield 79.5%.
¹H-NMR(CDCl₃) :δ1. 42(s, 9H), 3.08 (m, 2H), .3.71 (s, 3H), 4.66(s, 2H), 4.98(m, 1H), 7.11(d, 2H), 7.30(m, 2H)
MS(ESI): 309.9 [M+H]⁺

### Industrial Applicability

According to the present invention, optically active hydroxymethyl-substituted phenylalanine can be conveniently obtained optically selectively in a high yield. Moreover, according to the production method of the present invention, optically active hydroxymethyl-substituted phenylalanine can be produced industrially. In addition, a compound useful for the production method and a production method thereof are provided.

While some of the embodiments of the present invention have been described in detail in the above, those of ordinary skill in the art can enter various modifications and changes to the particular embodiments shown without substantially departing from the novel teaching and advantages of the present invention. Such modifications and changes are encompassed in the spirit and scope of the present invention as set forth in the appended claims.

This application is based on application No. 2005-084850 (filing date: March 23, 2005) filed in Japan, the contents of which are incorporated hereinto by reference.

## Claims

1. A method of producing optically active hydroxymethyl-substituted phenylalanine comprising reducing cyano-substituted benzylidene hydantoin represented by the following formula (1) 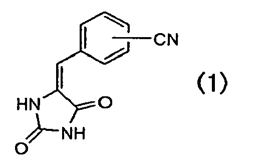 to give aminomethyl-substituted benzyl hydantoin represented by the following formula (2) 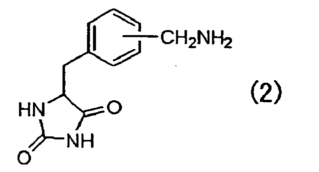 or a salt thereof, converting an amino group of the aminomethyl-substituted benzyl hydantoin or a salt thereof to a hydroxyl group to give hydroxymethyl-substituted benzyl hydantoin represented by the following formula (3) 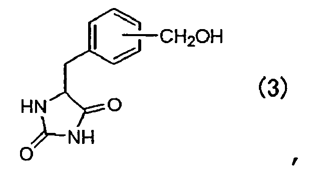 and treating the hydroxymethyl-substituted benzylhydantoin with an enzyme to give D-hydroxymethyl-substituted phenylalanine represented by the following formula (4a) 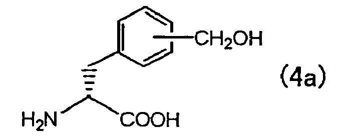 or a salt thereof, or L-hydroxymethyl-substituted phenylalanine represented by the following formula (4b) 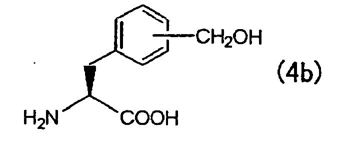 or a salt thereof.

2. The method of claim 1, wherein the reduction is performed by hydrogenation in the presence of a reduction catalyst.

3. The method of claim 2, wherein the reduction catalyst is a transition metal catalyst.

4. The method of claim 2, wherein the reduction catalyst is a palladium catalyst.

5. The method of any one of claims 1 to 4, wherein the amino group is converted to a hydroxyl group by reaction with a nitrite salt.

6. The method of any one of claims 1 to 5, wherein the enzyme is at least one kind selected from the group consisting of hydantoin racemase, hydantoinase and carbamoylase.

7. The method of any one of claims 1 to 5, wherein the enzyme is at least one kind selected from the group consisting of hydantoin racemase derived from Microbacterium liquefaciens AJ3912 strain, hydantoinase derived from Flavobacterium sp. AJ11199 strain and carbamoylase derived from Flavobacterium sp. AJ11199 strain.

8. The method of any one of claims 1 to 5, wherein the enzyme is obtained from a culture of a microorganism transformed with a recombinant DNA.

9. A method of producing aminomethyl-substituted benzyl hydantoin represented by the following formula (2) 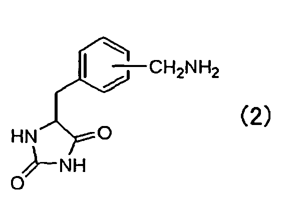 or a salt thereof, which comprises reducing cyano-substituted benzylidene hydantoin represented by the following formula (1)

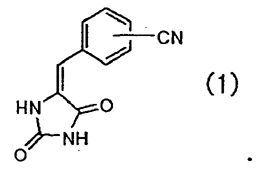

10. The method of claim 9, wherein the reduction is performed by hydrogenation in the presence of a reduction catalyst.

11. The method of claim 10, wherein the reduction catalyst is a transition metal catalyst.

12. The method of claim 10, wherein the reduction catalyst is a palladium catalyst.

13. A method of producing hydroxymethyl-substituted benzyl hydantoin represented by the following formula (3) 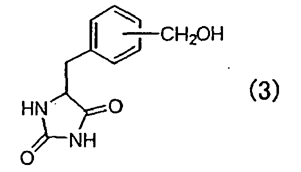 which comprises converting an amino group of aminomethyl-substituted benzyl hydantoin represented by the following formula (2) 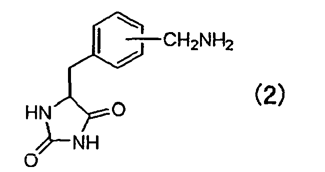 or a salt thereof to a hydroxyl group.

14. The method of claim 13, wherein the amino group is converted to a hydroxyl group by reaction with a nitrite salt.

15. A method of producing optically active hydroxymethyl-substituted phenylalanine, which comprises treating hydroxymethyl-substituted benzyl hydantoin represented by the following formula (3) 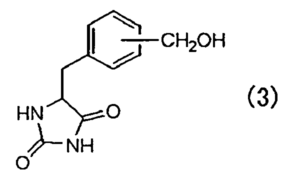 with an enzyme to give D-hydroxymethyl-substituted phenylalanine represented by the following formula (4a) 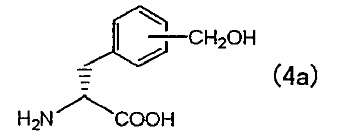 or a salt thereof, or L-hydroxymethyl-substituted phenylalanine represented by the following formula (4b) 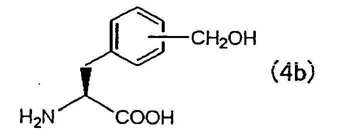 or a salt thereof.

16. The method of claim 15, wherein the enzyme is at least one kind selected from the group consisting of hydantoin racemase, hydantoinase and carbamoylase.

17. The method of claim 15, wherein the enzyme is at least one kind selected from the group consisting of hydantoin racemase derived from Microbacterium liquefaciens AJ3912 strain, hydantoinase derived from Flavobacterium sp. AJ11199 strain and carbamoylase derived from Flavobacterium sp. AJ11199 strain.

18. The method of claim 15, wherein the enzyme is obtained from a culture of a microorganism transformed with a recombinant DNA.

19. Aminomethyl-substituted benzyl hydantoin represented by the following formula (2) 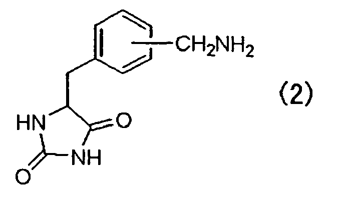 or a salt thereof.

20. Hydroxymethyl-substituted benzyl hydantoin represented by the following formula (3)


SEQUENCE LISTING

<110>  AJINOMOTO CO., INC.

<120>  PRODUCTION METHOD OF OPTICALLY ACTIVE HYDROXYMETHYL SUBSTITUTION  PHENYLALANINE

<130>  09887

<150>  JP 2005-084850
<151>  2005-03-23

<160>  5

<170>  PatentIn version 3.2

<210>  1
<211>  32
<212>  DNA
<213>  Artificial Sequence

<220>
<223>  Synthesised Primer

<400>  1
cgggaattcc atatgttcga cgtcattgtg aa                                32

<210>  2
<211>  29
<212>  DNA
<213>  Artificial Sequence

<220>
<223>  Synthesised Primer

<400>  2
cgcggatcct cacgacgaca cctcgctgt                                    29

<210>  3
<211>  30
<212>  DNA
<213>  Artificial Sequence

<220>
<223>  Synthesised Primer

<400>  3
caagttcacg taataaggag gtcgcatatg                                   30

<210>  4
<211>  32
<212>  DNA
<213>  Artificial Sequence

<220>
<223>  Synthesised Primer

<400>  4
cgggaattcc atatgacgct gcagcaggcg cg                                32

<210>  5
<211>  36
<212>  DNA
<213>  Artificial Sequence

<220>
<223>  Synthesised Primer

<400>  5
cggaattcgc ggatcctcac cggtcaagcg ccgtca                            36